# EUROPEAN PATENT APPLICATION

(11) **EP 4 571 593 A1**
(43) Date of publication of application: **18.06.2025**
(21) Application number: 24217121.3
(22) Date of filing: 03.12.2024
(51) Int. Cl.: G06N 3/098, G02C 13/00, G06T 11/60, G06Q 30/0601

(54) **FEDERATED LEARNING IN SERVICE ENVIRONMENTS**

(30) Priority: 14.12.2023 EP 23307206
(71) Applicant: Essilor International, 94220 Charenton-Le-Pont (FR)
(72) Inventor: LORE, Marie, 75020 PARIS (FR); LE CAIN, Aurélie, 94000 CRETEIL (FR); DEBIEUVRE, Amandine, 75012 PARIS (FR); GUEGAN, Julien, 75011 PARIS (FR); WONG, Yee Ling, SINGAPORE (SG); SAHLER, Jean, 92160 ANTONY (FR)
(74) Representative: Plasseraud IP

(57) **Abstract**

The disclosure relates to a computing device for an application in a retail environment, comprising:
an interface module configured to transmit and receive signals between a local neural network and a global neural network,
wherein:
the local neural network, implemented by the computing device, is configured to process and analyze in-shop data related to wearers or prospective wearers of head-worn devices for predictive and personalized service provision, and
the interface module enables the participation of the local neural network in a federated learning process with the global neural network through the transmitted and received signals.

## Description

### DOMAIN

The present invention relates to data science and more particularly to a computing device, a corresponding method and a corresponding computer-readable storage medium for deploying applications, for instance in a retail environment.

### BACKGROUND

In retail environments, there is a growing demand for advanced innovations and algorithms to provide personalized services to customers. However, the sharing of sensitive customer data and the need to comply with data protection regulations pose significant challenges. Opticians and other eyecare professionals (ECP) require access to algorithms and predictive models without compromising data privacy or violating confidentiality requirements.

Traditional approaches involve centralizing and sharing large volumes of data to improve predictive models and algorithms. Yet, this method is often impeded by regulatory restrictions, data protection rules, and the proprietary nature of customer information. There is a need for a novel solution that overcomes these limitations and enables retailers and self-employed individuals to leverage advanced algorithms while maintaining data privacy and confidentiality.

The existing approaches in the field of predictive modeling and machine learning have several drawbacks. The increasing regulation of sensitive data and the emphasis on customer data protection restrict the sharing of data with organizations, limiting ECP access to advancements that could enhance service quality.

Additionally, the lack of collective experience across different shops hinders the effectiveness of individual retailers' predictive models, which heavily rely on their own shop's experience or dataset.

Furthermore, the practical difficulties associated with collecting a large volume of data, such as compliance with data protection regulations and data storage limitations, pose obstacles to improving machine learning models.

There is a need for devices and methods that enable overcoming the above limitations.

### SUMMARY

The invention is defined by the appended independent claims. Additional features and advantages of the concepts herein disclosed are set forth in the description which follows.

The present disclosure aims at improving the situation.

To this end, the present disclosure describes a computing device, comprising:
an interface module configured to transmit and receive signals between a local neural network and a global neural network,
wherein:
   the local neural network, implemented by the computing device, is configured to process and analyze data related to wearers or prospective wearers of head-wearable devices for predictive and personalized service provision, and
   the interface module enables the participation of the local neural network in a federated learning process with the global neural network through the transmitted and received signals.

In other words:
the computing device is configured to, using the local neural network, process and analyze the data related to wearers or prospective wearers of head-wearable devices for the predictive and personalized service provision, and
the interface module is configured to transmit and receive the signals between the local neural network and the global neural network, thereby enabling the participation of the local neural network in the federated learning process with the global neural network through the transmitted and received signals.

For instance, the computing device may be a computing device for an application in a retail environment, comprising:
an interface module configured to transmit and receive signals between a local neural network and a global neural network,
wherein:
   the local neural network, implemented by the computing device, is configured to process and analyze in-shop data related to wearers or prospective wearers of head-worn devices for predictive and personalized service provision, and
   the interface module enables the participation of the local neural network in a federated learning process with the global neural network through the transmitted and received signals.

Federated learning offers a promising solution to these challenges. By utilizing a distributed and non-centralized data science technique, federated learning enables different entities to jointly train machine learning models without directly sharing data. This approach limits data flows, addresses ecological and security concerns, and allows for the utilization of more efficient and generalized algorithms.

The present disclosure also describes a method comprising:
collecting data related to wearers or prospective wearers of head-wearable devices by an input module of a computing device,
processing the in-shop data by a local neural network within the computing device, for the provision of at least one predictive and personalized service,
transmitting and receiving signals between the local neural network and a global neural network by an interface module within the computing device,
wherein the local neural network participates in a federated learning process with the global neural network facilitated by the transmission and reception of the signals.

For instance, the method may be a method for deploying an application in a retail environment, comprising:
collecting in-shop data related to wearers or prospective wearers of head-worn devices by an input module of a computing device,
processing the in-shop data by a local neural network within the computing device, for the provision of at least one predictive and personalized service,
transmitting and receiving signals between the local neural network and a global neural network by an interface module within the computing device,
wherein the local neural network participates in a federated learning process with the global neural network facilitated by the transmission and reception of the signals.

The present disclosure also describes a computer-readable storage medium, optionally a non-transitory computer-readable storage medium, having stored thereon a computer program comprising instructions which, when executed by a processor, cause the processor to carry out the method hereby described.

The storage medium may include hard drives, solid-state drives, CDs, USB drives, etc. In this context, it is being used to refer to whatever medium is storing the computer program that runs the method.

The present disclosure also describes a computer program comprising instructions that are accessible to a processor and which, when executed by the processor, cause the processor to carry out the method hereby described.

The present disclosure also describes a device equipped with a processor operably connected to a memory and to a communication interface, the device being configured to carry out any of the methods hereby described.

In an example, the data (or in-shop data) comprises images captured by an imaging device, depicting wearers or prospective wearers during physical try-ons of head-wearable (or head-worn) devices, and processing the data (or in-shop data) comprises determining boxing points corresponding to contours of the head-worn devices in the images, with the federated learning process enhancing the determination of the boxing points.

The processing of images (or in-shop images) to determine boxing points has practical applications in ensuring optimal fit for head-wearable (or head-worn) devices. This approach allows for precise customization, enhancing wearer comfort and satisfaction. The use of federated learning in this context includes improved accuracy in measurements, leading to better fitting products. It can be particularly useful in eyewear fitting, where even minor discrepancies can affect wearer comfort.

In an example, the data (or in-shop data) comprises real-time or static facial data of a prospective wearer, and processing the data (or in-shop data) comprises generating a simulated appearance of the prospective wearer with a head-wearable (or head-worn) device based on the facial data, implementing a virtual try-on process, with the federated learning process enhancing a rendering of the simulated appearance.

Utilizing real-time or static facial data to generate simulated appearances offers a compelling advantage for virtual try-ons. It enables customers to visualize how different head-wearable (or head-worn) devices will look on them without the need for physical try-ons, saving time and enhancing the shopping experience. In this context, federated learning allows for a more engaging and accurate representation of products, which is especially advantageous in fashion and eyewear retail.

In an example, the data (or in-shop data) further comprises feedback collected about the virtual try-on's perceived comfort from the prospective wearer by the input module, and processing the data (or in-shop data) further comprises adjusting the rendering of the simulated appearance by the local neural network in subsequent virtual try-ons based on the feedback.

Incorporating feedback into the virtual try-on process allows for iterative improvements, enhancing user satisfaction. By adjusting the rendering based on wearer feedback, the model can offer more personalized and comfortable experiences. This approach is particularly effective in tailoring virtual try-ons to individual preferences, leading to higher customer engagement and improved product selection.

In an example, the data (or in-shop data) comprises one or more measurements or characteristics of at least an eye of a prospective wearer, and processing the data (or in-shop data) comprises analyzing the one or more measurements or characteristics to determine a prescription for the prospective wearer, with the federated learning process enhancing the accuracy of the prescription.

Analyzing eye measurements or characteristics to determine prescriptions may contribute significantly to the recommendation process of personalized eyecare. Enhanced by federated learning, this method offers greater accuracy in prescription determination, with reduced errors and ultimately more tailored optical solutions for individuals.

In an example, the federated learning process includes merging or combining weights of a specific model from the local neural network with weights of other specific models of other local neural networks to create a generic model leveraging specific characteristics from all specific models, and updating the local neural network based on weights of the generic model, enhancing the local neural network's capabilities.

The merging or combining of model weights in the federated learning process fosters a more robust and adaptable neural network. By leveraging the strengths of various local models, the resulting generic model exhibits enhanced predictive power and versatility. This is particularly beneficial in diverse retail environments, where the ability to cater to a wide range of customer needs and preferences is crucial.

In an example, the local neural network facilitates continuous learning based on corrections or feedback associated with previous outputs of the local neural network.

The continuous learning capability of the local neural network, informed by feedback and corrections, ensures ongoing refinement and relevance. This results in a system that not only adapts to current trends and preferences but also improves its accuracy over time. This aspect is particularly advantageous in dynamic retail settings, where customer preferences and product offerings frequently evolve.

In an example, the data (or in-shop data) also includes wearer-specific information such as personal data, Internet of Things data, wearer preferences, and the local neural network leverages the wearer-specific information to enhance the accuracy and personalization of the service provision.

Leveraging wearer-specific information such as personal data and preferences enhances the personalization and accuracy of service provision. This approach enables a more tailored shopping experience, directly addressing the unique needs and preferences of each wearer. In environments such as bespoke eyewear or personalized health device retail, this capability can significantly elevate customer satisfaction.

In an example, the data (or in-shop data) comprise characteristics of a sightedness impairment control solution used by the wearer or prospective wearer and the predictive and personalized service provision comprises determining future values of vision characteristic of the wearer or prospective wearer.

In an example, the sightedness impairment is myopia.

In an example, the data (or in-shop data) comprise characteristics of an audio impairment control solution used by the wearer or prospective wearer and the predictive and personalized service provision comprises determining future values of a hearing characteristic of the wearer or prospective wearer.

### BRIEF DESCRIPTION OF THE DRAWINGS

Figure 1 depicts a general overview of a system adapted to contribute to the deployment of an application in a retail environment for head-worn devices, in an exemplary embodiment.
Figure 2 depicts a general structure of an artificial neural network, in an exemplary embodiment.
Figure 3 depicts a communication scheme between the elements represented on Figure 1, in an exemplary embodiment.
Figures 4 to 6 depict, in an exemplary embodiment, how federated learning can be used to simulate various scenarios of treatment efficacy for myopia control.

### DETAILED DESCRIPTION

The present disclosure is focused on methods and systems that contribute to the deployment of an application in a retail environment for head-worn devices.

"Retail environment" may include not just physical retail environments such as stores, experience centers, trade shows, expositions, service and support centers but also virtual or augmented reality spaces, online stores, and mobile commerce platforms.

"Head-wearable devices" may be any device that is wearable on the head. In the context of the present disclosure, this could include but not be limited to, eyeglasses, headsets, helmets, virtual reality devices, augmented reality devices, holographic displays, direct neural interfaces and so on. The head-wearable device may comprise one or more optical lenses. An optical lens is a piece of transparent substance with curved sides for concentrating or dispersing light.

By "retail environment for head-worn devices", it is understood a retail environment where head-worn devices are or may be sold, displayed or experienced by customers.

"Application" refers to a computer program, a service or more generally a piece of software that performs one or more functions for an end user or for another application. The term "application" should also include software suites, mobile apps, web-based platforms, and any future forms of software technology not yet developed. It can be standalone or part of a larger system.

"Deployment" should be understood to include the initial setup and/or the ongoing operation, maintenance, and upgrading of the application. This could encompass cloud-based services, in-store servers, or distributed computing environments. Deployment may involve integration with existing retail management systems, point-of-sale systems, or e-commerce platforms. This might include virtual try-on apps, customer relationship management (CRM) systems, or inventory tracking applications.

The main elements disclosed therein relate to a computing device or system which is suitable to participate in the deployment of the application in the retail environment.

The denomination "computing device or system" encompasses a wide range of computing hardware and may also designate virtual computing machines.

The computing device or system comprises, at least, an interface module configured to support bidirectional communication as both a signal transmitter and a signal receiver, by relying on any kind of data transmission technology, between a local neural network and a global neural network.

The signals encapsulate data that is relevant for facilitating operation of the local neural network in cooperation with the global neural network and/or for facilitating operation of the global neural network in cooperation with the local neural network. More specifically, the interface module is configured to enable the participation of the local neural network in a federated learning process with the global neural network through the transmitted and received signals.

The "local neural network" is associated with a local area of the network, be it from a geographical or topological standpoint, whereas the "global neural network" is associated with the entirety of the network or, at least, a plurality of local areas of the network. The distinction between the "local neural network" and the "global neural network" should be made fluid, allowing for various configurations and hierarchies of networks. For instance, a local network could be specific to a store, while a global network might span an entire retail chain or even include networks from different companies participating in data sharing for mutual benefit.

The computing device is further configured to implement, handle or manage the local neural network.

In its dual-function role, the computing device not only implements, manages, and handles the local neural network but also, through its interface module, acts as a pivotal communication link. The interface module specifically serves as a bridge facilitating interactions between the computing device and another external entity, such as another computing device or system. This external entity is tasked with implementing, managing, or handling the global neural network. Thus, while the computing device directly engages in the intricate processing and administration of the local neural network, its interface module extends its functionality by enabling seamless, bidirectional communication with the global neural network, thereby reinforcing the computing device's integral role in a broader interconnected system.

The computing device or system may further comprise a non-transitory storage medium which stores, at least, data contained in a signal to be transmitted by the interface module and/or in a signal that has been received by the interface module. The non-transitory storage medium may further store additional data not contained in any signal to be transmitted using the interface module and not contained in any signal having been received using the interface module.

The computing device or system may further comprise a processing unit configured to at least contribute in managing the local neural network, in coordination with the non-transitory storage medium and with the interface module.

The computing device or system may further comprise other elements including, without being limited to, input modules and/or output modules. A human-machine interface is an example of interface that may serve as an input module and/or as an output module.

Examples of computing devices or systems in the context of the present disclosure include a terminal in a shop or store, a gateway device in that shop or store and/or a server coordinating data across a local group of shops or stores.

By implementing the local neural network, the computing device or system processes and analyzes data related to wearers or prospective wearers of head-worn devices for predictive and personalized service provision.

"data" is data associated to one or more locations among a larger group of locations. The locations may be, for instance, shops, for example, physical stores or virtual shops allowed to sell products on an online marketplace, ECP offices, hospitals, or the house of the user when the user is collecting data or using the data directly in-house. The " data" may encompass data generated within the physical confines of a shop and/or data related to the shop's operations, which could be generated online or through other channels. This can include online customer reviews, virtual fitting room data, or social media interactions related to the shop's products. By being related to wearers or prospective wearers of head-worn devices, the in-shop data has a logical link with one or more persons wearing, or interested in wearing, a head-worn device. The in-shop data may refer to a broad range of data including one or more of the following: biometric data such as head size and shape, interpupillary distance, eye tracking data, vision acuity tests results, prescription data, eye health assessments, usage patterns, preference data, performance data, purchase history, demographic data, feedback data, interaction data, environmental or contextual data, technological compatibility data, behavioral data such as non-verbal cues, etc.

The "data" may also comprise data related to values of parameters of sightedness impairment control solution that is used or usable by the wearer.

The sightedness impairment control solution may be chosen among one or more of the following elements:
- myopia control spectacle lenses,
- myopia control contact lenses,
- orthokeratology contact lenses,
- a treatment using atropine,
- a treatment using red light therapy and
- no use of the sightedness impairment control solution.

The "data" may also comprise data related to values of parameters of audio impairment control solution that is used or usable by the wearer.

The audio impairment control may be chosen among one or more of the following elements:
- hearing aids,
- cochlear implants,
- bone-anchored hearing systems,
- assistive listening devices
- personal sound amplification products,
- tinnitus maskers,
- audiologic rehabilitation programs,
- environmental sound amplifiers,
- hearing protections with augmented features such as selective amplification and/or noise-canceling features.

In the context of the present disclosure, the application utilizes, or leverages, a prediction output by the local neural network for a wearer or prospective wearer of a head-worn device to provide a personalized service. For instance, the personalized service may be guidance for an end user, such as the wearer or prospective wearer or such as an eye care practitioner or may be an input for another in-shop application aimed at the end user. Examples of services include fitting optimization, virtual try-on enhancement and vision/hearing characteristics forecasting.

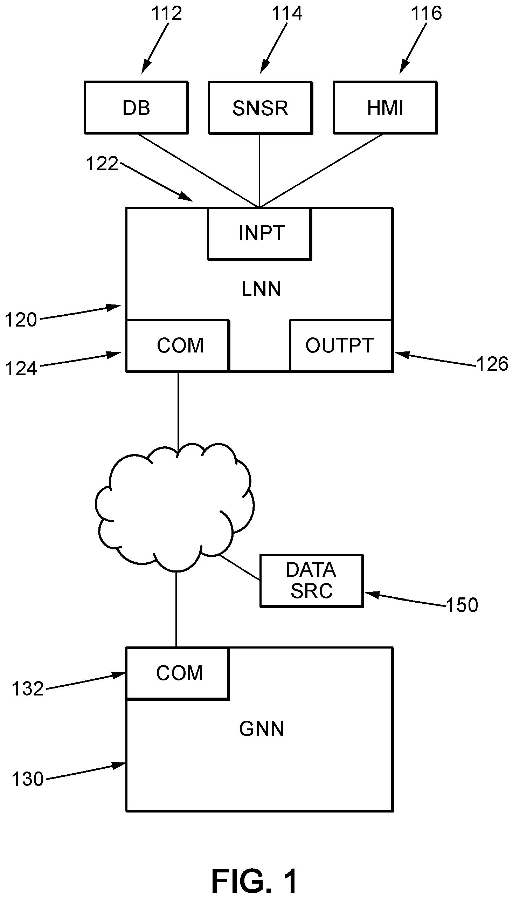

It is now referred to Fig. 1, which depicts an exemplary computing system according to the present disclosure.

The computing system comprises a local computing device or system (120) implementing a local neural network and further comprises a central computing device or system (130) implementing a global neural network. The central computing device or system may be one or more remote servers. For instance, the global neural network may be implemented as an application in a cloud-based environment. The global neural network may be configured to communicate with other data sources (150), like other local neural networks, cloud databases storing anonymized data from multiple wearers or external APIs providing real-time data on weather.

The local computing device or system and the central computing device or system are configured to communicate with each other using a communication channel or a communication link. This communication link is symbolically represented by a pair of interface modules (124, 132).

The local computing device or system further comprises an input module (122) configured to obtain, receive, collect or access in-shop data from one or more data sources such as, for instance, one or more databases (112), one or more sensors (114) and/or one or more human machine interfaces (116) while the local neural network employs this data to build a local model. The input module is designed to accommodate a diverse range of inputs from the data sources.

A sensor, or sensing module, is a device or part of a device that is able to detect and respond to some type of input from the physical environment. In the context of the present disclosure, the sensors may include one or more cameras to capture images of the wearer or prospective wearer or of the environment. The sensors may further include ambient light sensors for light condition analysis, proximity sensors for nearby object detection, accelerometers to measure direction and speed of head movement, eye trackers, in particular infrared eye trackers, to identify gazing direction, time of flight sensors to sense gazing distance, etc.

A human machine interface is a device or software that allows humans to interact with machines. In the context of the present disclosure, human machine interfaces may be adapted to receive for instance tactile inputs or auditory inputs such as voice commands. Other examples of suitable human machine interfaces include gesture recognition systems and graphical user interfaces on connected devices. Some human-machine interfaces may allow, for instance, providing declarative data about the wearer, such as their age, gender, laterality, facial features, prescription, etc. or retrieving compiled data on a frame of a head-worn device, termed "frame boxing data", all of which may be provided as inputs to the system.

The local computing device or system further comprises an output module (126) configured to output or provide predictions by processing the in-shop data using the local model.

A possible use case of the local neural network is for fitting optimization. Fitting optimization involves determining at least an outline of a spectacle frame on a wearer as appearing in an input image, so as to derive from said outline at least one fitting parameter of the spectacle frame on said wearer. The above wording "fitting parameter" is to be interpreted in a broad sense. It can aim typically a position of a specific part of the spectacle frame relatively to the wearer's face such as, for example, the spectacle bridge on a nasal part of the wearer, and/or a spectacle branch position relatively to a temple of the wearer, etc. It can aim also fitting parameters in optometric field such as a pupillary distance, fitting heights, eye rotation center position, pantoscopic angle, wrap angle, eye-lens distance and/or eye rotation center-lens distance, etc., when the wearer wears said spectacle frame. The outline of a spectacle frame is also typically called "bounding box". Outlining the frame (and/or lenses in the frame) is tedious for the ECP. Errors, or at least inaccuracies so as to go faster, can be made and the quality of determination of fitting parameters can be poor then. For this reason, it is advantageous to perform an automatic "frame detection" process, which involves processing an image where the frame is visible to identify the location and shape of the frame. Some known frame detection processes involve artificial intelligence means trained to provide a bounding box as an output when an image of the wearer wearing the spectacle frame is provided as input. In the use case according to the present disclosure, a combination of high-resolution cameras, 3D scanners and accelerometers may be used as sensors (114) to provide in-shop data. These sensors may collect detailed images of the wearer's face, precise measurements of head shape and size, and head movement patterns. Using these sensors, digital measurement tools and human-machine interfaces (116), eye care practitioners (ECP) may take pictures and modify lens boxing points in view of fitting lenses to the face of wearers or prospective wearers. Databases (112) may store the pictures, the lens boxing points, wearer-specific data, ECP-specific data, etc. The in-shop data from the abovementioned data sources is provided as input to the local model which may be trained to process the in-shop data in order to provide lens boxing points as output. As a result, the output of the local model may be used to automatically adjust and detect fitting measurements, optimizing physical and virtual frame fittings, and/or in order to identify design issues, contributing to a richer lens configuration model. The local model may learn from the adjustments performed by the ECP to better determine the lens boxing points, potentially factoring in other in-shop data such as wearer-specific data such as faces of each individual wearer or prospective wearer, shop-specific data such as lighting conditions and spatial sensor arrangements, ECP-specific data indicating practices of each individual ECP, etc.

Another possible use case of the local neural network is for virtual try-on enhancement. In this use case, cameras and environmental sensors in kiosks may be used to collect, as in-shop data, user images of wearers or prospective wearers. Lighting, contrast and backgrounds may be varying from a kiosk to another, meaning that the in-shop data of different kiosks have systematic deviations. Each kiosk may further provide a virtual try-on application which is configured to use the user images of a specific user, being a wearer or prospective wearer, to generate virtual views of the user with synthetic frames. The local model may then learn from the user images collected, potentially adjusting for image characteristics like contrast and luminosity.

Another possible use case of the local neural network is for determining at least one future value of at least one vision characteristic of the wearer depending on the type and the value of characteristics of a sightedness impairment control solution used by the wearer.

The vision characteristic of the wearer may be any one of the following elements:
- a spherical error of a right eye or a left eye of the wearer,
- a binocular spherical equivalent of the right eye or the left eye,
- a binocular spherical equivalent of the right eye or the left eye,
- a refraction of the right eye or the left eye,
- a prescription of the right eye or the left eye,
- an axial length of the left eye or the right eye,
- an average of the axial length of the left eye and the axial length of the right eye,
- a corneal parameter of the left eye or the right eye,
- an indication of a presence of myopia on the left eye or the right eye, and
- a variation, during a period of time, of at least one among the spherical error, the monocular spherical equivalent, the binocular spherical equivalent, the refraction, the prescription, the axial length, the corneal parameter or the indication.

Another possible use case of the local neural network is for determining at least one future value of at least one hearing characteristic of the wearer depending on the type and the value of characteristics of an audio impairment control solution used by the wearer.

The audio characteristic of the wearer may be any one of the following elements:
a hearing threshold level
a speech discrimination score,
a frequency-specific hearing loss,
a dynamic range,
a tinnitus characteristic
a difference between a bone-conduction level and an air-conduction level,
a loudness growth, and
a hearing aid usage pattern.

In these cases one may also consider at least one physiological parameter of the wearer. This physiological parameter may be:
an age of the at least one model wearer or the wearer,
a gender of the at least one model wearer or the wearer,
an ethnicity of the at least one model wearer or the wearer,
a location of the at least one model wearer or the wearer,
a number of myopic parents of the at least one model wearer or the wearer,
a duration of near work realized by the at least one model wearer or the wearer and
a duration of time spent outdoors by the at least one model wearer or the wearer.

All these embodiments illustrated in the previously described use-cases may be combined, for instance an image of a virtual frame may be output as a replacement for an image of a real spectacle frame worn by the wearer on an input picture of the wearer. To do so, several consecutive method steps may be considered: identifying an outline of the real spectacle frame worn by the wearer in said input picture, determining anchor points of the real spectacle frame on the wearer's face, and using said anchor points to place, in the input picture, an image of the virtual frame. This scenario illustrates the possibility of training the local neural network for a simple individual task or for a single combined task. For instance, the local neural network may be trained for only identifying an outline of the real spectacle frame worn by the wearer in said input picture as in the first mentioned use case, or for only using anchor points as further input data to place, in the input picture, an image of the virtual frame. The latter however requires the anchor points to be determined in a preprocessing step, either fully automatically by any appropriate technical means or through human-machine interaction. Alternatively, assuming a sufficient amount of training data and computing resources, the local neural network may be trained to directly generate an output picture corresponding to the input picture and with the image of the virtual frame in place of the real frame in a "black box" fashion, without distinctly identifying the outline of the spectacle frame or determining anchor points of the real spectacle frame on the wearer's face.

The global neural network, while operating in a similar architecture to the local neural network, is configured to utilize a distinct set of data inputs (150) for building its global model. Notably, this includes historical models built using aggregate data similar to that collected by the input modules of various local computing devices or systems. This aggregate data represents a cumulation of insights drawn from multiple local models, each generated from in-shop data within their respective retail environments.

The local neural network participates in a federated learning process with the global neural network, allowing both neural networks to refine and update their respective models. Federated learning is a machine learning approach where a model is trained across multiple decentralized devices or servers holding local data samples, without exchanging them. This approach maintains data privacy and reduces the need for data centralization.

Optionally, the global neural network may be interconnected with a multitude of local neural networks, functioning as a central node in a broader networked ecosystem. In this configuration, the global neural network could initially be one of the local neural networks, which has evolved or been appointed to serve as the global network. This unique position allows it to harness and synthesize diverse data patterns and trends from across the network, enhancing its predictive capabilities.

By leveraging this network-wide data, the global neural network can refine and update its global model, benefiting from a wide-ranging perspective that encompasses varied retail environments and consumer interactions. This approach ensures that the learning process is enriched by a diverse and comprehensive dataset, while adhering to the principles of federated learning where actual data samples are not exchanged, thus maintaining individual data privacy and reducing the necessity for centralized data storage.

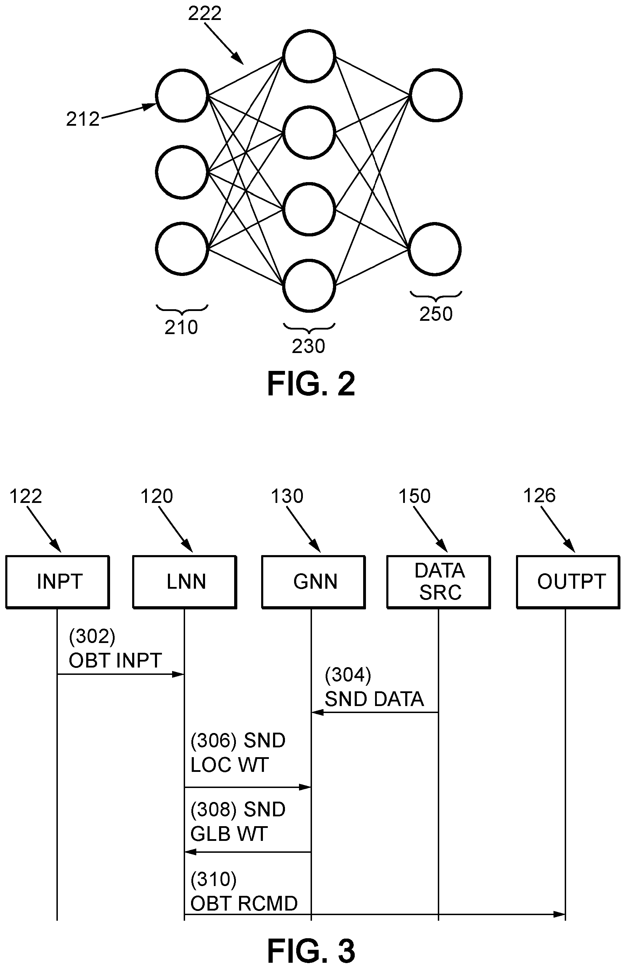

Reference is now made to Figure 2 which provides a general structure of an artificial neural network, in an exemplary embodiment. This general structure is applicable both to the local neural network and to the global neural network. It is a simplified representation, aiming to convey the key elements of the network structure, rather than an accurate depiction of the network's complexity in real-world applications.

The illustrated artificial neural network comprises multiple layers of neurons. At the ends, there's an input layer (210) and an output layer (250), with one or more hidden layers (230) sandwiched between them. Each of these layers hosts several neurons (212), interconnected with every neuron in the adjacent layer. These interconnections are referred to as "weights".

An artificial neural network is generally used to create and adjust models. A training phase involves providing labelled training data as inputs to the input layer. In machine learning, the "training data" refers to the raw data that the model learns from, and the "labels" refer to the corresponding outcomes or classes that the model is trained to "predict". The weights are numerical parameters in the neural network that are adjusted during the training phase to improve the predictions output by the output layer. After the training phase is complete, the artificial neural network enters production phase, to receive as inputs raw data that is not necessarily labelled and to output corresponding predictions.

For illustrative clarity, Figure 2 only displays a few layers and a handful of neurons. In practice, neural networks incorporate significantly more layers, densely populated with a vast number of interconnected neurons, creating a myriad of weights or connections that extend far beyond what is feasibly representable in a single diagram.

In this disclosure, the term "local weight" refers to a connection between two neurons within the local neural network, while a "global weight" analogously refers to a connection within the global neural network. Generally, there's a one-to-one correspondence between local and global weights, implying that the structure of the local neural network (including the number of layers, the number of neurons within each layer, and the function of each neuron) mirrors that of the global neural network. However, it should be noted that this one-to-one correspondence isn't strictly necessary for the operation of the system, as long as the local and global networks share a compatible structure that allows the mapping or transformation of global weights into the local network context.

Reference is now made to Figure 3 which provides a communication scheme between the elements represented on Figure 1, in an exemplary embodiment.

It is considered that, at a given instant, a variety of in-shop data, including specific in-shop data relevant for a particular wearer or prospective wearer, has been obtained (302) by the input module (122) as a result of one or more interactions with the one or more human-machine interfaces, or as a result of one or more queries to the one or more databases, or from the regular functioning of the one or more sensors.

The obtained in-shop data is transmitted to a processing unit of the local computing device or system (120) and processed using the local model. This means in particular that in-shop data that is associated to a particular person (wearer or prospective wearer) is fed as input to the input layer (210) of the local neural network. As a result of processing the obtained in-shop data using the local model, a personalized recommendation for the particular wearer or prospective wearer is obtained (310) by the output module (126). This means that, as a result of feeding the in-shop data associated to a particular person as input to the input layer (210) of the local neural network, the output layer (210) of the local neural network generates a personalized recommendation for the particular person.

The in-shop data and the personalized recommendation may then be both associated to the particular person and considered, from the standpoint of the local model, as forming a set of labelled data akin to the labelled training data. In this case, the personalized recommendation is the label.

The local model may then continuously learn when provided with new in-shop data and providing new personalized recommendations, and as a result of the continuous learning the local weights may continuously evolve.

To better understand the inputs and outputs in the context of this system, we can refer to the two use cases already mentioned.

In the use case relating to fitting optimization, the inputs might include detailed images of the wearer's face, measurements of head shape and size, head movement patterns, lens boxing points as adjusted by an ECP on captured pictures of the wearer's face etc. The outputs might include optimized lens boxing points, adjusted fitting measurements, or identified design issues.

In the use case relating to virtual try-on, the inputs might include user images of wearers or prospective wearers, data derived from these user images such as physical attributes and environmental conditions. The inputs might further include other in-shop data, for instance user-related data such as age, prescription, user preferences, wearing patterns, activity patterns, etc. The outputs might include renderings such as virtual try-on images that are adjusted for some image characteristics like contrast and luminosity.

In both use cases, the inputs primarily consist of detailed in-shop data related to the wearers' physical attributes and environmental conditions, captured through various sensors and interfaces. The outputs are tailored recommendations and enhancements - in the case of fitting optimization, it's precise lens boxing points and fitting measurements; for virtual try-on, it's the generation of realistic virtual views with synthetic frames, adjusted for environmental variables.

The present disclosure is however not limited to these specific use cases.

More generally, the local model may admit as inputs many types of in-shop data related to wearers or prospective wearers, that may be provided in various formats (as variables, as text, as images, as audio signals, as video signals, etc.) and which are descriptive of information including, but not limited to:
user demographics and preferences such as age, gender, and occupation of the wearer, personal style preferences (modern, classic, sporty, etc.), preferred frame materials (metal, plastic, composite, etc.), or color preferences for frames and lenses,
optical measurements and eye health data such as prescription details (spherical, cylindrical power, axis, etc.), pupillary distance and other ocular measurements, information about any specific eye conditions (astigmatism, presbyopia, myopia, etc.), or previous eyewear prescriptions and their comfort levels,
lifestyle and usage data such as information about the wearer's daily activities and environments (outdoors, office work, exposure to screens, etc.), specific needs like UV protection, anti-reflective coatings, or blue light filtering, or sports or other activity-based requirements,
biometric and physiological data such as head shape and ear-to-nose measurements, skin tone, eye color, and facial features, or any allergies or sensitivities to certain materials,
historical data and feedback, such as previous purchases and feedback on them, changes in prescription over time, or wearers' history of adjustments and repairs.

The local model may admit as inputs many types of in-shop data that is not related to a specific wearer or prospective wearer but rather shop-specific. Such shop-specific data may be descriptive of environmental factors, equipment and technology specifications, operational practices, shop layout and design, ambient conditions, digital and virtual interface settings, etc.

The outputs of the model may include various types of recommendations that may be provided in various formats (as variables, as text, as images, as audio signals, as video signals, etc.) and which are descriptive of information including, but not limited to:
frame recommendations such as suggested frame styles that match the wearer's personal style, face shape, and color preferences, or recommendations for frame materials based on skin sensitivities and lifestyle needs,
lens type and feature suggestions, such as specific lens types or settings (single vision, bifocal, progressive, etc.) based on prescription and age, recommendations for lens coatings and treatments (anti-reflective, scratch-resistant, UV protective coatings), or suggested tint colors and intensity based on usage (for instance, photochromic lenses for outdoor use),
customized eyewear solutions, including tailored recommendations for eyewear suited to specific activities or occupations (e.g., computer glasses, sports eyewear), solutions for complex prescriptions or unique eye conditions, or adaptive recommendations based on changes in prescription or eye health over time,
interactive features such as virtual try-on renderings with recommended frames and lenses, or simulations showing a visual difference with various lens treatments,
health and/or comfort optimization, such as suggestions for eyewear that minimizes eye strain or fatigue, especially important for screen-heavy lifestyles, or recommendations for eyewear that caters to specific eye health conditions.

Since some wearer-specific inputs may be affected by shop-specific conditions, providing in-shop data descriptive of the shop-specific conditions as inputs to the local model allows the model to adjust output personalized recommendations accordingly.

For instance, in-shop data related to wearers or prospective wearers originates from a specific, limited group of wearers or prospective wearers. This group predominantly comprises individuals who have either visited or engaged with the shop, be it in a physical or virtual setting. The data thus reflects the experiences, preferences, and interactions of a distinct subset of customers, providing a focused and relevant basis for the local neural network's training and subsequent recommendations.

Similarly, in-shop data related to ECP originates from a single ECP or a small group of ECPs working in or for the shop.

Similarly, in-shop data originates from one or more sensors located in the shop, with potential systematic bias. For instance, when a sensor is a camera, several factors may contribute to this systematic bias, such as lighting conditions, camera quality and definition, camera angle and positioning, distance of subjects from the camera, shop layout and design, color calibration and image processing settings, etc.

In order to enhance the personalized recommendations provided to wearers or prospective wearers, it is proposed to generalize the local model across a larger cohort of wearers or prospective wearers.

This generalization can be performed asynchronously, for instance concurrently or periodically. Alternatively, this generalization can be performed according to a predetermined scheme in relation to obtaining the in-shop data, for example every time an amount of new in-shop data exceeding a predetermined threshold is obtained since the last performed generalization.

This generalization process involves a sequence of elementary actions which is now described in an exemplary scenario where a plurality of local computing devices or systems (120) each implement a corresponding local neural network which corresponds to a specific shop and where each local computing device or system is configured to communicate with a same central computing device or system (130).

First, the weights inferred from the local neural networks, encapsulating the correlations between the amassed training data and their respective labels, are respectively transmitted (306) by the corresponding local computing device or system (120) to the central computing device or system (130) implementing the global neural network. The central computing device or system (130) may also receive (304) pertinent data from other sources (150), such as a centralized database storing demographic information about the wearers, environmental data, or information gleaned from other devices in the network.

A generalized model is formulated by the central computing device or system (130). This may involve selecting a subgroup of shops or local neural networks for the generalization step, and computing an average of the neural network weights from the selected shops or local neural networks to establish a global neural network model. Based on federated learning (FL) principles, this model symbolizes the collective intelligence of the chosen shops or local neural networks.

Subsequently, the global weights from the global model are relayed back (308) to the individual local computing devices or systems.

Upon receipt, these global weights are integrated into the local model, thusly forming an updated local model. This may involve selectively replacing certain local weights while retaining others. Various methods can be utilized for this, such as transmitting only the global weights designated to replace local ones, or delivering a broader set of global weights and employing a filtering mechanism at the local neural network module to select specific weights for replacement. Either way, the revised local model constitutes a personalized adaptation of the generalized model, preserving aspects that are unique to the wearer while integrating insights from the global neural network.

Ultimately, the personalized recommendation (310) may be determined based on the updated local model. This approach allows each local neural network to remain adapted to specific local in-shop data while dynamically adapting to new situations, leveraging the collective learning amassed from a broader set of wearers or prospective wearers.

The disclosure further introduces enhancements to eye condition management, utilizing federated learning to improve the predictability and efficacy of interventions tailored to individual needs.

The field of eye condition management encompasses a wide range of evolutive eye conditions such as myopia, hyperopia, astigmatism, and presbyopia. Management strategies are decided by eye care practitioners (ECPs) and often involve interventions aimed at controlling or mitigating the progression of these conditions for specific individuals.

Known myopia control management tools have been provided as assistance to help ECPs decide on specific management strategies for specific individuals. These known myopia control management tools utilize various strategies to showcase the potential benefits of controlling myopia. These tools typically present a patient's myopia progression over time by plotting the evolution of spherical equivalent based on standard myopia control efficacy rates derived from literature. However, these techniques may not be fully suited for recurring decision-making due to their reliance on static efficacy rates that do not consider individual patient variability and do not adapt to changes over time. Moreover, the aggregation of data in centralized systems for these predictions poses risks to privacy and limits the dynamic updating of treatment plans.

It is further known to employ machine learning algorithms for predicting the progression of eye conditions based on a variety of inputs such as refractive status, age, gender, and other demographic or environmental factors. These predictive models use longitudinal and cross-sectional data to forecast changes in eye conditions and can be applied using various classical machine learning techniques. For example, some methods use linear prediction models like Support Vector Regression (SVR) and Gaussian Process Regression (GPR), which analyze data collected from various sources to predict outcomes. These techniques, however, require the aggregation of potentially sensitive data in a central location, which can lead to concerns about data privacy and are susceptible to breaches. Moreover, such methods often do not allow for real-time updates or easy incorporation of new data without comprehensive reprocessing, which limits their practicality for ongoing patient care.

The disclosure herein explores the transformative method of federated learning for eye care practitioners (ECPs) to predict the effectiveness of various myopia control interventions. Unlike traditional centralized data processing methods, federated learning enables the secure processing of data across a network of devices, allowing for collective insights without compromising patient privacy.

This involves a predictive analysis of treatment efficacy using federated networks, ensuring that sensitive data remains within local devices. The application of this approach allows for dynamic adjustments based on real-time data from multiple users, enhancing the adaptability and accuracy of myopia management programs.

Federated learning not only secures patient data but also enhances the management programs by integrating new data points from ongoing patient interactions and treatments.

While the primary focus has been on myopia, the principles of federated learning can be broadly applied to other eye conditions. The versatility of federated learning allows for its adaptation to conditions where tailored interventions can significantly benefit from predictive modeling.

The predictive capabilities of federated learning are employed to assess the efficacy of interventions across a spectrum of eye conditions, involving the adjustment of treatments based on predicted progression rates or the responsiveness of a patient to certain therapies.

As federated learning continuously integrates new data, it facilitates the dynamic adjustment of treatment plans, such as quickly adapting interventions for presbyopia if initial strategies show suboptimal efficacy.

By utilizing a network that learns from diverse data inputs, federated learning supports a more personalized approach to eye care, optimizing resource utilization and enhancing patient outcomes.

Additionally, federated learning supports the development of decision-making tools that help practitioners choose the most effective treatment plans based on predictive models that simulate various outcomes.

The integration of federated learning models with existing electronic health record (EHR) systems ensures seamless access to predictive insights during patient consultations, enhancing the decision-making process for single and/or combination interventions.

The general description of how federated learning is implemented in a retail environment, as illustrated in Figures 1 to 3, also applies similarly to any service environment and in particular to an eye health service environment.

When federated learning is implemented in an eye health service environment, the same steps of collecting data, processing and analyzing data, and network communication between a local neural network and a global neural network are implemented.

The differences only concern the nature of the data that is collected, processed, analyzed and transmitted.

The data collection involves acquiring specific data related to an eye condition of one eye or of both eyes of an individual through an input module of a computing device. This data can include, but is not limited to, biometric data, health metrics, environmental exposure, and historical health records.

The collected data is processed and analyzed by a local neural network within the computing device. This local processing allows for the dynamic adaptation of the model based on local data inputs without compromising the privacy of the individual's data.

The local neural network communicates with a global neural network via an interface module. This communication involves the transmission and reception of signals that facilitate a federated learning process, allowing for the enhancement of predictive accuracy through collective learning from diverse data sources.

Finally, the service provided is predictive and personalized and utilizes the analyzed data to forecast the efficacy of candidate treatments for specific condition of the individual's eye, thereby aiding healthcare providers in making informed decisions.

Specific examples of data related to eye conditions may include, but are not limited to, one or more, or a combination, of the following elements: age, prescription, visual acuity measurements, intraocular pressure readings, retinal scans, corneal topography data, patient-reported symptoms, historical treatment responses, genetic information related to eye health, environmental and lifestyle data.

The data related to eye condition may include immediate or recent measurements and observations such as recent visual acuity measurements indicating the current clarity of vision.

The data related to eye condition may include historical data or long-term data that track the evolution of one or more eye conditions over time, for instance the progression fo prescription changes over years.

The data related to eye condition, whether current and/or historical, forms a multidimensional array that feeds into the local neural network

Specific examples of candidate treatments may include, but are not limited to, one or more, or a combination, of the following elements: pharmacological treatments such as eye drops for glaucoma, surgical interventions like LASIK or cataract surgery, wearable vision correction devices like glasses or contact lenses, light-based therapies such as photodynamic therapy, regenerative medicine approaches like stem cell injections.

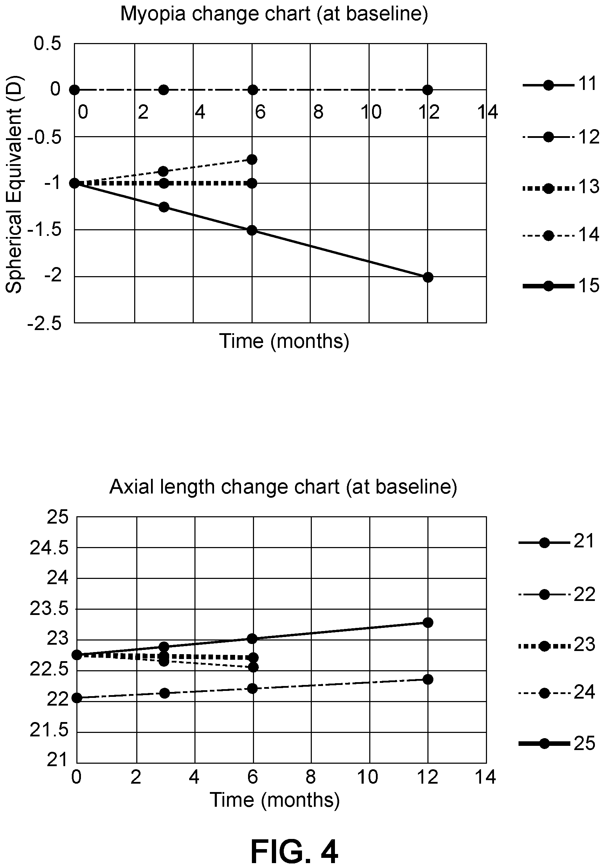

An exemplary embodiment is now described in relation to Figures 4 to 6. In this embodiment, the eye condition of the individual is myopia, and the efficiency of the candidate treatment is evaluated based on its ability to control the evolution of myopia over time.

In the context of myopia management, data collected may include, but are not limited to, one or more, or a combination, of the following elements: historical progression of myopia in diopters over time, measurements from autorefractors or similar devices to track changes in refractive error, lifestyle data such as time spent on near work versus outdoor activities, genetic factors that may predispose individuals to myopia, responses to previous myopia control treatments, such as atropine use or orthokeratology.

Candidate treatments specifically for myopia might involve, but are not limited to, one or more, or a combination, of the following elements: prescription of corrective lenses with specific optical properties to slow progression, application of low-dose (e.g. 0.01%) atropine eye drops, use of orthokeratology lenses worn overnight to reshape the cornea, recommendations for increased outdoor activities based on epidemiological data.

Figures 4 to 6 illustrate these concepts by showing how federated learning can be used to simulate various scenarios of treatment efficacy for myopia control, based on the integration of diverse data inputs. The figures depict how different treatment strategies might alter the progression of myopia over time, providing a visual and quantifiable method for ECPs to assess the potential benefits of each treatment option and make adjustments based on real-time data feedback.

Figure 4 depicts, for an eye of an individual:
measures of a spherical equivalent 15 (in diopters) and an axial length 25 (in mm) at a first time t = 0 months,
predicted values at 3, 6 and 12 months from the first time t of a spherical equivalent 11 and an axial length 21 in a hypothesis where myopia is not corrected and no myopia control strategy is applied,
predicted values at 3, 6 and 12 months from the first time t of a spherical equivalent 12 and an axial length 22 in a hypothesis where myopia is corrected by prescribing corrective lenses and no myopia control strategy is applied,
predicted values at 3, 6 and 12 months from the first time t of a spherical equivalent 13 and an axial length 23 in a hypothesis where a first myopia control strategy is applied,
predicted values at 3, 6 and 12 months from the first time t of a spherical equivalent 14 and an axial length 24 in a hypothesis where a second myopia control strategy is applied.

For instance, both the first and the second myopia control strategies involve the prescription of corrective lenses with specific optical properties to slow progression, and the second myopia control strategy further involves the prescription of 0.01% atropine eye drops.

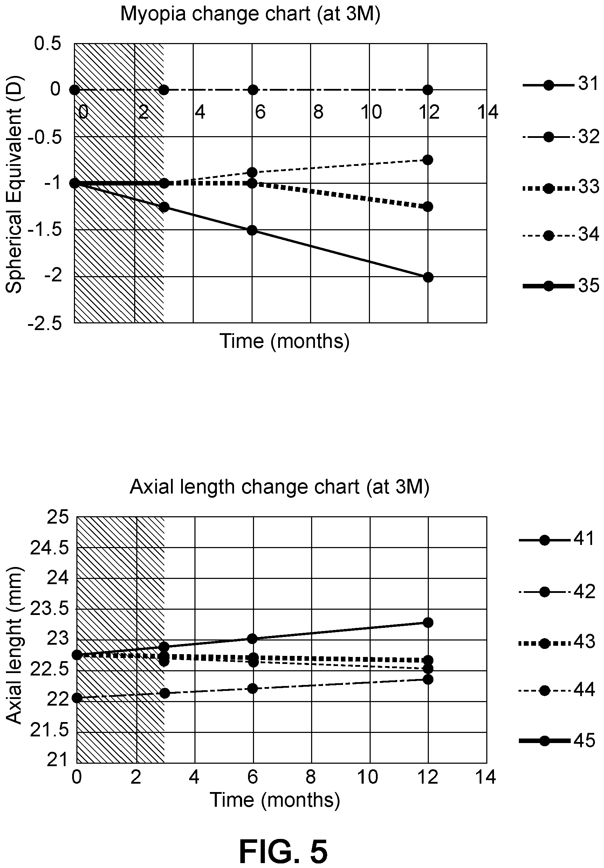

Figure 5 depicts, for the eye of the individual and after the first myopia control strategy has been applied between the first time t and a second time t' = 3 months after the first time t:
measures of a spherical equivalent 35 (in diopters) and an axial length 45 (in mm) at the first time t and at the second time t',
the predicted values at 3, 6 and 12 months from the first time t of a spherical equivalent 31 and an axial length 41 in the same hypothesis where myopia is corrected and no myopia control strategy is applied as in Figure 4,
the predicted values at 3, 6 and 12 months from the first time t of a spherical equivalent 32 and an axial length 42 in the same hypothesis where myopia is corrected by prescribing corrective lenses and no myopia control strategy is applied as in Figure 4,
updated predicted values at 6 and 12 months from the first time t of a spherical equivalent 33 and an axial length 43 in a hypothesis where after the first myopia control strategy has been applied between the first time t and the second time t', the first myopia control strategy continues to be applied after the second time t',
updated predicted values at 6 and 12 months from the first time t of a spherical equivalent 34 and an axial length 44 in a hypothesis where after the first myopia control strategy has been applied between the first time t and the second time t', the second myopia control strategy is applied after the second time t'.

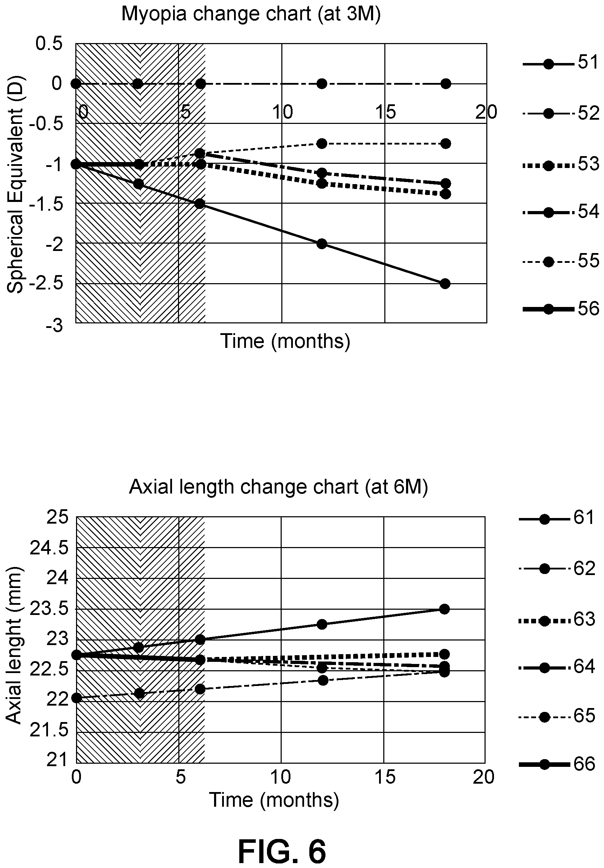

Figure 6 depicts, for the eye of the individual, after the first myopia control strategy has been applied between the first time t and the second time t' = 3 months after the first time t and after the second myopia control strategy has been applied between the second time t' and a third time t" = 6 months after the first time t:
measures of a spherical equivalent 56 (in diopters) and an axial length 66 (in mm) at the first time t, at the second time t' and at the third time t",
the predicted values at 12 and 18 months from the first time t of a spherical equivalent 51 and an axial length 61 in the same hypothesis where myopia is corrected and no myopia control strategy is applied as in Figures 4 and 5,
the predicted values at 12 and 18 months from the first time t of a spherical equivalent 52 and an axial length 62 in the same hypothesis where myopia is corrected by prescribing corrective lenses and no myopia control strategy is applied as in Figures 4 and 5,
updated predicted values at 12 and 18 months from the first time t of a spherical equivalent 53 and an axial length 63 in a hypothesis where after the first myopia control strategy has been applied between the first time t and the second time t' and after the first myopia control strategy has been applied between the second time t' and the third time t", the first myopia control strategy continues to be applied after the third time t",
updated predicted values at 12 and 18 months from the first time t of a spherical equivalent 54 and an axial length 64 in a hypothesis where after the first myopia control strategy has been applied between the first time t and the second time t' and after the second myopia control strategy has been applied between the second time t' and the third time t", the first myopia control strategy is applied after the third time t", and
updated predicted values at 12 and 18 months from the first time t of a spherical equivalent 55 and an axial length 65 in a hypothesis where after the first myopia control strategy has been applied between the first time t and the second time t' and after the second myopia control strategy has been applied between the second time t' and the third time t", the second myopia control strategy continues to be applied after the third time t".

This general description is intended to present an exemplary implementation of the invention. Variations, modifications, and alternatives may be apparent to those skilled in the art and can be made without departing from the scope of the invention. The specific configuration of components and the manner in which they interact are merely illustrative, and alternative configurations and interactions are within the scope of the appended claims.

In light of the general description, the following specific embodiments serve to further illustrate the proposed invention. These embodiments correspond to distinct use-cases, each presenting a unique approach towards personalized smart eyewear operation. Each embodiment encompasses the same general process already depicted in Figure 3, which involves data collection, model creation, model generalization, and the practical application of the model to various functionalities in the form of personalized recommendations.

## Claims

1. A computing device, comprising:
an interface module configured to transmit and receive signals between a local neural network and a global neural network,
wherein:
the local neural network, implemented by the computing device, is configured to process and analyze data related to wearers or prospective wearers of head-wearable devices for predictive and personalized service provision, and
the interface module enables the participation of the local neural network in a federated learning process with the global neural network through the transmitted and received signals.

2. A method comprising:
collecting data related to wearers or prospective wearers of head-wearable devices by an input module of a computing device,
processing the in-shop data by a local neural network within the computing device, for the provision of at least one predictive and personalized service,
transmitting and receiving signals between the local neural network and a global neural network by an interface module within the computing device,
wherein the local neural network participates in a federated learning process with the global neural network facilitated by the transmission and reception of the signals.

3. The method of claim 2, wherein:
the data comprises images captured by an imaging device, depicting wearers or
prospective wearers during physical try-ons of head-wearable devices,
processing the data comprises determining boxing points corresponding to contours of the head-wearable devices in the images, with the federated learning process enhancing the determination of the boxing points.

4. The method of claim 2, wherein:
the data comprises real-time or static facial data of a prospective wearer,
processing the data comprises generating a simulated appearance of the prospective wearer with a head-wearable device based on the facial data, implementing a virtual try-on process, with the federated learning process enhancing a rendering of the simulated appearance.

5. The method of claim 4, wherein:
the data further comprises feedback collected about the virtual try-on's perceived comfort from the prospective wearer by the input module, and
processing the data further comprises adjusting the rendering of the simulated appearance by the local neural network in subsequent virtual try-ons based on the feedback.

6. The method of claim 2, wherein:
the in-shop data comprises one or more measurements or characteristics of at least an eye of a prospective wearer, and
processing the data comprises analyzing the one or more measurements or
characteristics to determine a prescription for the prospective wearer, with the federated learning process enhancing the accuracy of the prescription.

7. The method of any one of claims 2 to 6, wherein the federated learning process includes:
merging or combining weights of a specific model from the local neural network with weights of other specific models of other local neural networks to create a generic model leveraging specific characteristics from all specific models, and
updating the local neural network based on weights of the generic model, enhancing the local neural network's capabilities.

8. The method of any one of claims 2 to 7, wherein the local neural network facilitates continuous learning based on corrections or feedback associated with previous outputs of the local neural network.

9. The method of any one of claims 2 to 8, wherein the data also includes wearer-specific information such as personal data, Internet of Things data, wearer preferences, and the local neural network leverages the wearer-specific information to enhance the accuracy and personalization of the service provision.

10. A non-transitory computer-readable storage medium having stored thereon a computer program comprising instructions which, when executed by a processor, cause the processor to carry out the method according to any one of claims 2 to 9.

11. The computing device of claim 1, wherein the data related to wearers or prospective wearers of head-wearable devices comprise in-shop data.

12. The computing device of claim 1 or 11, wherein the data comprise characteristics of a sightedness impairment control solution used by the wearer or prospective wearer and the predictive and personalized service provision comprises determining future values of vision characteristic of the wearer or prospective wearer.

13. The computing device of claim 12, wherein the sightedness impairment is myopia.

14. The computing device of any one of claims 1 and 11 to 13, wherein the data comprise characteristics of an audio impairment control solution used by the wearer or prospective wearer and the predictive and personalized service provision comprises determining future values of a hearing characteristic of the wearer or prospective wearer.
